# EUROPEAN PATENT APPLICATION

(11) **EP 4 809 986 A1**
(43) Date of publication of application: **23.09.2026**
(21) Application number: 26163547.8
(22) Date of filing: 10.03.2026
(51) Int. Cl.: A61F 2/30

(54) **DEVICE AND METHOD FOR MAKING A PROSTHESIS COVERED IN MEDICAL CEMENT**

(30) Priority: 17.03.2025 IT 202500005370
(71) Applicant: G21 S.r.l., 41039 San Possidonio (MO) (IT)
(72) Inventor: FORONI, Filippo, 41037 Mirandola (MODENA) (IT); FORONI, Maurizio, 41037 Mirandola (MODENA) (IT)
(74) Representative: Gagliardelli, Fabrizio

(57) **Abstract**

Described is a method for making a covered articulated prosthesis (10) in an operating room, comprising the steps of:
Preparing an articular prosthesis (11) designed to replace, partly or completely, the functionality of a joint;
Providing a covering chamber (3);
Positioning at least one portion of the articular prosthesis (11) inside the covering chamber (3);
Introducing a quantity of a substance inside the covering chamber (3), covering the portion of articular prosthesis (11);
Waiting for a predetermined time for the solidification of the substance injected into the covering chamber (3), creating a covering around the stretch of articular prosthesis (11) positioned in the covering chamber (3) to obtain the covered articular prosthesis;
Releasing the covered articular prosthesis from the covering chamber (3) at the end of the predetermined time for the solidification;
Discarding the covering chamber (3) after releasing the covered articular prosthesis.

## Description

The present invention relates to the field of orthopaedic surgery.

In particular, the present invention relates to the field of making implantable devices in the operating room, in particular temporary implantable devices in replacement of infected prostheses, to allow the healing of the respective implant site.

The object of the present invention is a method for making a covered articular prosthesis in the operating room.

Further objects of the present invention are a device for making in an operating room a covered articular prosthesis according to said method and a kit, comprising said device and the covered articular prosthesis, made according to said method.

Moulds are known in the sector for making temporary implantable devices in the operating room; in particular, moulds are known for making femoral components for hip spacers in the operating room.

The devices made by means of prior art moulds are obtained by covering a prosthesis with a layer of medical cement. In the case of hip spacers, therefore, the femoral component is obtained by covering a femoral stem with a layer of medical cement.

The devices thus made are intended to be implanted in place of an infected prosthesis and are kept at the implant site for a period of time corresponding to the time for healing from the infection. At the end of this period, the device is replaced with a new long-term prosthesis, performing the so-called revision protocol in two stages.

The prior art moulds are typically made of metallic material and comprise two half-shells, which can be coupled to internally identify a seat for positioning the medical cement and the prosthesis to be covered. Further, the two semi-shells, coupled together, define openings above for the introduction of the prosthesis, in order to make the covering with medical cement.

In detail, a quantity of medical cement in the form of paste, that is, mediumhigh viscosity fluid, is placed in the seat identified by the coupled half-shells. Subsequently, the prosthesis, uncovered, is introduced into the mould through the upper opening, until it reaches the correct position in the seat inside the mould.

Once the correct positioning has been reached, an end portion of the prosthesis protrudes from the mould through the upper opening. In the case of a femoral component, the end portion substantially corresponds to a femoral neck portion.

The end portion, in detail, allows during the insertion step to maintain the grip on the prosthesis, while the medical cement is distributed around the prosthesis inside the mould; the end portion also allows the prosthesis to be maintained in the correct position inside the mould, until the medical cement around the prosthesis has completely solidified.

At the end of the moulding step, the half-shells are uncoupled, releasing the implantable device comprising the covered prosthesis.

The prior art moulds, however, have clear limitations and can be improved overall.

Firstly, in order to allow the correct closure of the half-shells, a quantity of silicone is used, dispensed at the coupling edge of the half-shells, in order to seal and, consequently, correctly isolate the inner chamber, avoiding unwanted escape of cement through said edge.

Further, the prior art moulds, made of metallic material, must be subjected to one or more cleaning cycles using solvents, in order to remove any cement residue at the end of their use. Without careful cleaning, it would not be possible to guarantee adequate sterility during a subsequent implant of a device made using said moulds. For this purpose, the solvents might be applied several times and the washing and cleaning operations can be particularly long and laborious.

The same applies for the silicone used to seal the coupling edge of the half-shells: in fact, this must also be completely removed before subsequent use, eliminating all traces by means of appropriate solvents and thereby making the mould cleaning operations even more complex and costly.

The aim of the present invention is to provide a method for making a covered prosthesis in the operating room, a device for making a covered prothesis in the operating room following said method and a kit comprising said device, which is capable of overcoming the obvious limitations of the prior art. The aim of the present invention is also to provide a covered articular prosthesis made according to said method.

In particular, the technical purpose of the present invention is to provide a method for making a covered prosthesis in the operating room, which makes it possible to avoid the use of solvents and the lengthy cleaning operations described. Consequently, the technical purpose of the present invention is to provide a device for making a covered prosthesis in the operating room according to said method and a kit comprising said device, which is easier to use for an operator.

The technical purpose and the aims specified are achieved by a method for making in the operating room a covered articular prosthesis, a device for making in the operating room a covered prosthesis by means of said method, a kit comprising said device and the covered articular prosthesis, made by means of said method, comprising the technical characteristics set forth in one or more of the appended claims.

Further characteristics and advantages of the present invention will become clearer from the indicative, and therefore non-limiting, description of a preferred embodiment of a method for making a covered articular prostheses, a device for making a covered articular prostheses in the operating room following said method, a kit comprising said device, and the covered articular prostheses made by means of said device and by using said method.

This description is set out below with reference to the accompanying drawings which are provided solely for illustrative and therefore non-limiting purposes, in which:
- Figure 1 shows an isometric view of an embodiment of a device for making a covered prosthesis according to the present invention;
- Figure 2 shows an external side view of the device of Figure 1 in a first embodiment, in an open configuration;
- Figure 3 shows an internal side view of the device of Figure 2;
- Figure 4 shows an exploded view of the device of Figures 2 and 3 and a prosthesis intended to be covered;
- Figure 5 shows an exploded view of the device of Figures 2 and 3 and the prosthesis intended to be partially covered inserted in said device;
- Figures 6 shows an exploded view of the device of Figure 1 in a second embodiment, and a prosthesis intended to be covered;
- Figure 7 shows an exploded view of the device of Figure 1 in the second embodiment and the prosthesis intended to be partially covered, inserted in said device;
- Figure 8 shows a device for making a covered prosthesis, in an open configuration, in a further embodiment.

The first object of the present invention is a method for making a covered articular prosthesis in the operating room.

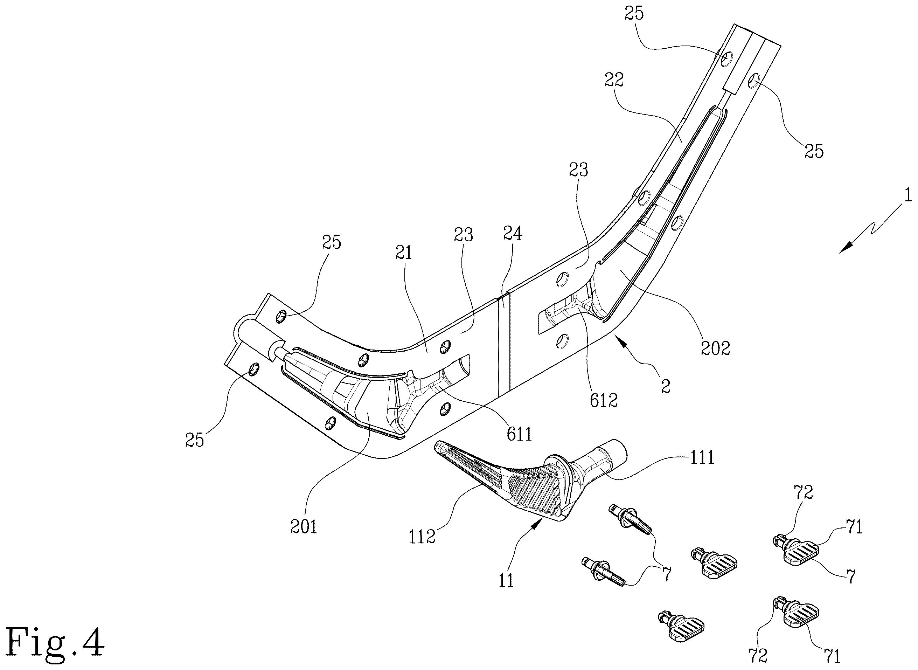

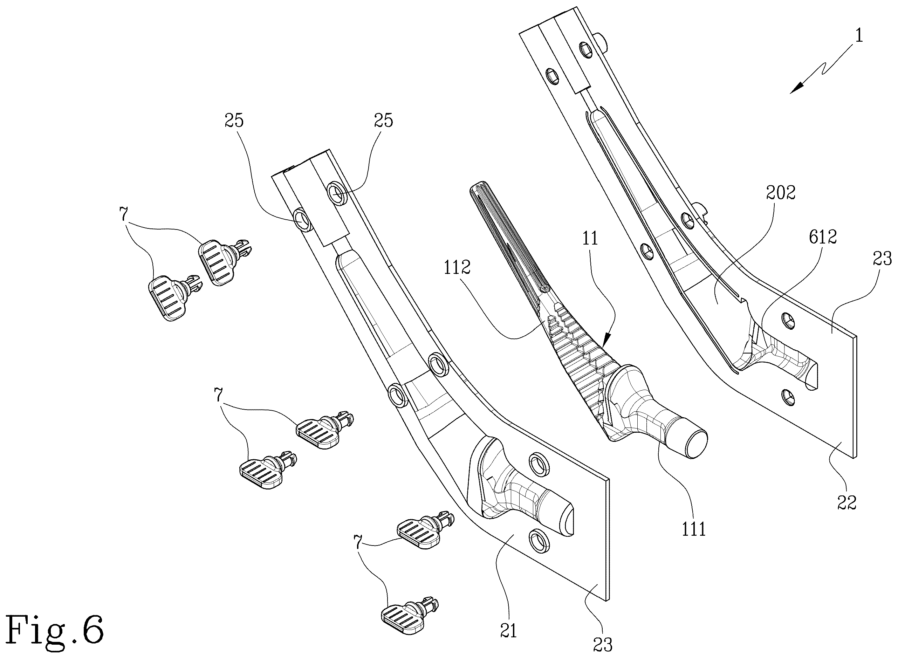

With references to Figures 4 and 6, the method according to the present invention comprises firstly the step of providing an articular prosthesis 11. The term "articular prosthesis" refers to an implantable medical device suitable for partially or completely replacing the functionality of a joint.

An articular prosthesis is intended to functionally replace a joint in the long term, that is, for a period of time potentially corresponding to the remaining life of the patient. An articular prosthesis is in use positioned at a functionally and structurally compromised joint, that is to say, in pathological conditions, in order to restore the functionality and anatomical structure corresponding to physiological conditions.

For this purpose, an articular prosthesis must have a sufficient resistance to stresses, in particular to cyclical stresses, to which a joint is typically subjected. In addition, an articular prosthesis must be made of biocompatible material, in order to limit inflammatory reaction and immune response by the patient's body and promote integration with the body itself. Materials that meet high stress resistance and sufficient biocompatibility requirements can comprise ceramic materials, such as alumina and zirconia, metallic materials, such as titanium and chromium-cobalt alloys, polymeric materials, such as high and ultra-high molecular weight polyethylene (HMWPE, UHMWPE), and combinations of the foregoing materials. Other materials and combinations of materials, known in the prior art, may form an articular prosthesis.

An example of prior art articular prostheses is complete hip prostheses comprising articulating femoral components with their respective acetabular cups. The femoral prosthesis is intended to be positioned at the proximal portion of the femur and inserted into the proximal femoral channel, while the acetabular cup is intended to be placed at the acetabulum of the pelvis. The femoral prosthesis typically has a femoral stem made of titanium or titanium alloy and a hemispherical head made of zirconia or zirconiaalumina, while the acetabular cup is typically made of titanium or titanium alloy.

The term "covered articular prothesis" refers to an implantable medical device comprising an articular prosthesis of the type specified in the preceding paragraphs, on the outer surface of which a covering comprising a substance is applied. The substance can be of various types and can, in use and once implanted, perform various functions.

In other words, the covering substance may interact in various ways with the tissues surrounding the implant zone. Further, the covering made with the substance of interest may partially or totally affect the external surface of the uncovered articular prosthesis.

For this reason, the method according to the present invention enables various types of articular prostheses to be obtained, partially or totally covered in a substance of interest, starting from the prior-art uncovered articular prostheses. For example, the method according to the present invention enables femoral stems to be obtained that are partially or fully covered with medical cement, starting from uncovered femoral stems 11, of known type, for example made of titanium or its alloys.

The substance used to make the covering may comprise, for example, medical cement. Preferably, the substance comprises medical cement with the addition of at least one antibiotic. The medical cement covering may, therefore, partially or totally affect the external surface of the uncovered articular prosthesis. For this reason, a covered articular prosthesis has a partial or total external covering of medical cement.

The articular prosthesis covered with antibiotic-loaded medical cement interacts with the implant site by treating a bacterial infection arising at the implant site or preventing the occurrence of a bacterial infection at said site. In this case, for example, the method according to the present invention allows femoral stems to be obtained which are partially or totally covered with medical cement, starting from uncovered femoral stems 11, of known type, for example made of titanium or its alloys. The femoral stem covered in medical cement supplemented with an antibiotic enables the weakening or prevention of a bacterial infection at the implant site, that is, at the hip joint.

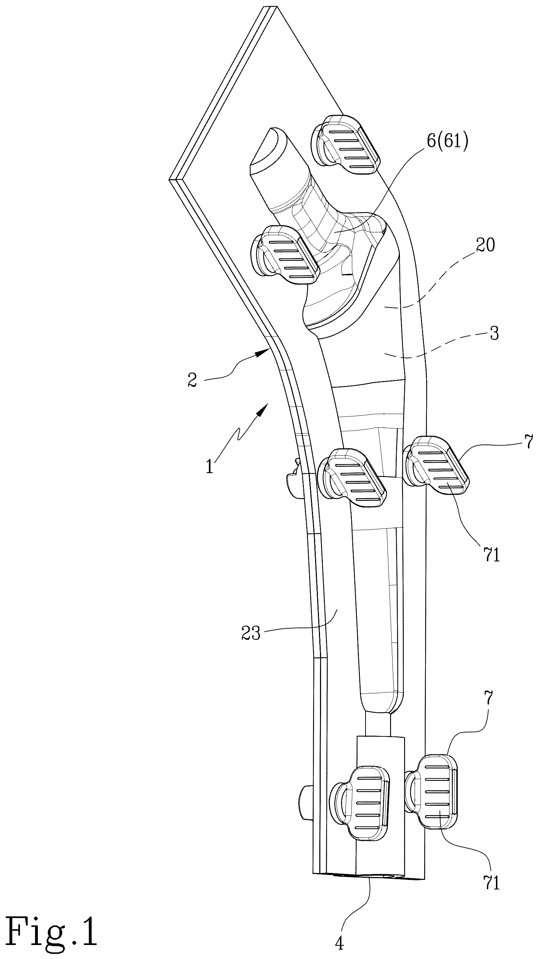

After the preparation of the articular prosthesis 11, the method comprises the step of providing a covering chamber 3 and the step of positioning at least one portion of the articular prosthesis 11 in the covering chamber 3 (Figure 1).

The covering chamber 3 is shaped as a cavity suitable for receiving the articular prosthesis 11, to allow its partial or total covering. For this reason, the covering chamber 3 preferably has a three-dimensional extension similar to that of the articular prosthesis 11, allowing it to be covered without substantially altering its external profile. The term "external profile" refers to the three-dimensional shape of the articular prosthesis 11 or of a section thereof, defined respectively by the entire external surface of the articular prothesis 11 or by a portion of said external surface.

Covering chambers 3 which are available in a wide range of shapes and sizes and suitable for receiving, in whole or in part, respective articular prostheses 11 of different types, can be used in the steps of the method according to the invention. For example, covering chambers 3 of elongated shape can be used for making a covered stem prosthesis: for example, with reference to Figure 1, a covering chamber 3 of elongated shape can be used for making a covered femoral stem or a covered humeral stem. Alternatively, covering chambers 3 having a pair of convexities to reproduce two femoral condyles may be used to make a covered femoral component of a knee prosthesis.

Further types of covering chambers 3 may be provided in the method according to the invention.

In order to make a covering on the articular prosthesis 11, thereby obtain the covered articular prosthesis, the method according to the present invention comprises introducing a quantity of a substance of interest into the covering chamber 3, covering at least a portion of the articular prosthesis 11 located in the covering chamber 3. The covering, in other words, comprises said substance of interest.

The substance can be introduced into the covering chamber 3 through a first opening 4 to put the covering chamber in communication with the outside.

The substance can be introduced into the covering chamber 3 by different methods.

Preferably, in the step of introducing a quantity of substance into the covering chamber 3, a quantity of substance is provided in liquid form and is introduced into the covering chamber 3 by injection through the first opening 4.

The substance, in liquid form, can then be injected into the covering chamber 3, in which the articular prosthesis 11 or a section thereof is positioned.

The substance, introduced into the covering chamber 3 or injected through the first opening 4, is distributed inside the covering chamber 3 to cover, that is, coat the articular prosthesis 11 or the stretch of articular prosthesis 11.

With reference to Figure 1, considering a covering chamber 3 of elongated shape for the covering of a stem prosthesis, the first opening 4 can be located at a distal end of the covering chamber 3. In other words, the injection of the substance can take place through the first opening 4 located in an end portion of the chamber, opposite a proximal part intended to receive the neck of the stem portion.

Considering, among the possible variants, a covering in medical cement, the medical cement can be introduced into the covering chamber 3 in different ways, preferably by injection through the first opening 4.

During the introduction of a quantity of medical cement into covering chamber 3 a quantity of medical cement is in fact prepared in liquid form and introduced into the covering chamber 3 by injection. An operator can prepare a cartridge filled with liquid medical cement, intended to be coupled to the first opening 4: the medical cement, in liquid form, can then be injected into the covering chamber 3, in which the articular prosthesis 11 or a section thereof is positioned.

The medical cement, introduced into the covering chamber 3 or injected through the first opening 4, is distributed inside the covering chamber 3 to cover, that is, coat the articular prosthesis 11 or the stretch of articular prosthesis 11.

In order to obtain the desired covering around the articular prosthesis 11, the step of ensuring the correct positioning of the articular prosthesis 11 or the portion of the articular prosthesis 11 inside the covering chamber 3 can be provided; preferably, the operation of making sure the correct positioning is carried out prior to the step of introducing an amount of substance inside the covering chamber 3.

The possible pressure exerted by the substance introduced into the covering chamber 3 could, in fact, cause displacement of the articular prosthesis 11 or a stretch of the articular prosthesis 11 inside the covering chamber 3, resulting in the covering not being distributed around the articular prosthesis 11 as desired.

By ensuring the positioning of the articular prosthesis 11 with respect to the covering chamber 3, the articular prosthesis 11 can, therefore, maintain the correct positioning inside the covering chamber 3 during the introduction of the substance: the covering thus obtained is distributed in a desired or expected manner around the articular prosthesis 11.

In detail, the correct positioning of the articular prosthesis 11 or of the portion of the articular prosthesis 11 inside the covering chamber 3 can be ensured and maintained by centring means 6 positioned at the covering chamber 3. Preferably, the positioning of the articular prosthesis 11 is ensured by engaging at least a stretch of the articular prosthesis 11 in a centring seat 6 at the covering chamber 3. A centring seat 6 can, for example, be made by means of a reduction in cross-section of the covering chamber 3: said centring seat 6 can therefore be engaged by a stretch of articular prosthesis 11 shaped in such a way as to match, to create a constraint or joint. The constraint formed at the centring seat 6 prevents the articular prosthesis 11 or the stretch of articular prosthesis 11 of interest from moving due to the introduction of the substance, for example the injection of the medical cement in liquid form, inside the covering chamber 3.

Preferably, the covering intended to be made around the articular prosthesis 11 has a uniform thickness, resulting from a homogeneous distribution of the substance in the covering chamber 3, around the articular prosthesis 11 or the portion of it located therein. It is, in fact, preferable to obtain a covered articular prosthesis 10 with a uniform covering of substantially constant thickness around the articular prosthesis 11 or the stretch of articular prosthesis 11, so as not to alter the overall three-dimensional shape of the articular prosthesis 11 which is to be covered.

For this reason, the centring means 6 or the centring seats 6 are such as to secure and keep the articular prosthesis 11 centred within the space identified by the covering chamber 3, so as to allow a homogeneous distribution of the medical cement around the articular prosthesis 11, thereby making a covered articular prosthesis having a covering of medical cement of homogeneous thickness.

In a variant of the present method, there are steps of providing an insert 12, which can be inserted or located inside the covering chamber 13, and positioning or locating the insert 12 in a desired position inside the covering chamber 3, modifying the shape or at least one internal dimension of the covering chamber 3.

This step is carried out, in particular, before the step for introducing the cement.

Thanks to the positioning of the insert 12, the cement fills the available covering chamber 3, that is, the covering chamber not engaged by the insert 12. For example, following the step for positioning the insert 12, the covering chamber 3 might be shorter or narrower, adapting the covering chamber 3 to specific manufacturing needs, without resorting to a different covering chamber 3.

Further, the method according to the invention provides the further step of waiting a predetermined time for the solidification of the substance injected into the covering chamber 3, making the covering from said substance of interest, in the solid state, around the articular prosthesis 11 or the section of articular prosthesis 11 positioned in the covering chamber 3 and obtaining the covered articular prosthesis.

At the end of the above-mentioned steps, the articular prosthesis 11 fully inserted into the covering chamber 3 is completely covered with the substance, resulting in a covered articular prosthesis 10 with a covering on its entire external surface.

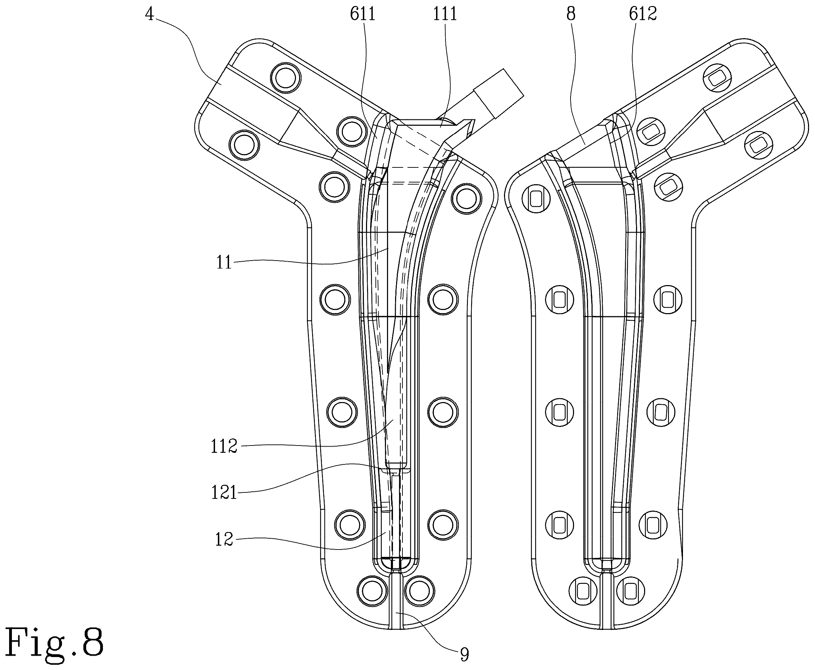

Alternatively, at the end of the above-mentioned steps, the articular prosthesis 11 partially inserted inside the covering chamber 3 is partially covered with the substance of interest, to make a covered articular prosthesis 10 having a covering at the portion of the external surface located inside the covering chamber 3 at the time of introduction of the substance. For example, with reference to Figure 8, the articular prosthesis 11 can be partially inserted into the covering chamber 3 and can have an end portion protruding from the covering chamber 3 through a second opening 8 to put the covering chamber 3 in communication with an external environment: in this case, the articular prosthesis 11 has the medical cement covering at its external surface, with the exception of the end portion, which, on the other hand, does not have the medical cement covering.

The method according to the invention therefore comprises the step of releasing the covered articular prosthesis from the covering chamber 3 at the end of the predetermined time for the solidification. In this way, the covered prosthesis is made available to an operator, who can then proceed to implant it in the articular site for which it is intended.

After being released, the covered articular prosthesis can be subjected to manual or mechanical machining. For example, the covered articular prosthesis can be subjected to one or more surface treatment steps, in order to obtain to achieve a desired finish suitable to meet particular implant requirements. In detail, burrs formed by excess substance or unwanted surface irregularities can be removed by abrasion.

The covered articular prosthesis made by the method according to this invention therefore contains internally the articular prosthesis 11, used to confer resistance to the stresses, in particular to the cyclical stresses to which the joint is subjected under physiological operating conditions; further, the covered articular prothesis is covered externally with a covering of the desired substance, intended to interact with the implant site according to specific needs.

Considering the medical cement, the covered articular prosthesis made by the method according to the invention therefore has internally the articular prosthesis 11 used to confer resistance to the stresses and externally has a covering of medical cement, preferably added with at least one antibiotic. In this embodiment, the method provides for the further step of adding a substance to the medical cement used for the covering, preferably an antibiotic substance suitable for weakening an infection in progress at the site of the implant. This step can be scheduled before the medical cement is introduced into the covering chamber 3, or afterwards, once the covering is complete.

The covered articular prosthesis having the covering 12 of medial cement with the addition of an antibiotic can be used as a replacement for an infected prosthesis previously implanted in a joint seat.

The covered articular prosthesis can then be placed in the articular seat and can be maintained there for at least as long as necessary to allow healing from the infection.

In a variant of the present method, the further step of positioning the covering chamber 3 at a support element may be provided, before or after the step of introducing the substance into the covering chamber 3. For example, the covering chamber 3 can be positioned at a support intended to support and maintain the covering chamber 3 in position to allow the correct solidification of the substance around the articular prosthesis 11. The positioning of the covering chamber 3 at said support further also means that, during the solidification time of the substance, the operator responsible for making the covered articular prosthesis does not use their hands to hold the articular prosthesis 11 or the covering chamber 3, but is free to carry out other operations in preparation for the implant of the covered articular prosthesis 10.

Finally, the method according to the present invention comprises the step of disposing of the covering chamber 3 after releasing the covered articular prosthesis. In other words, the covering chamber 3, once the covered articular prosthesis is made, is not used for making the covering of a further articular prosthesis 11, that is, it is not used for making a further covered articular prosthesis. The covering chamber 3 is not, therefore, reusable, that is to say, it is single-use. There are multiple methods by which the covering chamber 3 can be disposed of once the covered articular prosthesis has been released.

Thanks to the disposal of the covering chamber 3 at the end of use, the method according to the present invention enables covered articular prostheses to be obtained without the use of solvents to remove residues of the substance used for the covering from the covering chamber 3; in other words, the disposal of the covering chamber 3 at the end of use avoids the cleaning operations, which are often time-consuming, that precede a subsequent use.

The steps of the method according to the present invention can be completed by means of a device 1 for making a covered articular prosthesis in the operating room.

The device 1 for making a covered articular prosthesis in the operating room therefore constitutes a further object of the present invention.

With reference to the accompanying drawings, the reference numeral 1 denotes in its entirety the device for making a covered articular prosthesis. The device 1 comprises a shell 2 delimiting internally a covering chamber 3 for the positioning of at least one portion of an articular prosthesis 11 (Figure 1).

The shell 2 has an inner surface 20 delimiting the covering chamber 3 which is suitable for receiving the articular prosthesis 11 or a portion of the prosthesis 11, as well as a quantity of a substance selected for making a covering around the articular prosthesis 11 or the portion of the articular prosthesis 11 positioned therein.

In fact, the shell 2 has a first opening 4 for putting in communication the covering chamber 3 with an outside environment, to allow the introduction of medical cement inside the covering chamber 3 and to cover the at least one portion of articular prosthesis 11, obtaining the covered articular prosthesis .

In other words, the substance introduced into the covering chamber 3 is distributed around the articular prosthesis 11 and the inner surface 20 is intended to contain the substance introduced and to impart a predetermined shape to the substance introduced in the covering chamber 3, allowing the desired covering to be made around the articular prosthesis 11.

In one embodiment, the first opening 4 is an opening 4 for the injection of a liquid substance; in particular the first opening 4 is an injection opening 4 for a quantity of medical cement in liquid form, which can be coupled to a prior art cartridge containing said medical cement in liquid form.

The device 1 is, totally or partly, made of a single-use material.

The device 1 is, therefore, totally or partially not reusable. In the case of the device 1 being partially reusable, the device 1 comprises reusable parts or components that can be uncoupled at the end of use from the non-reusable parts.

Prior to an implant operation, the device 1 can therefore be supplied in a sterile condition and then used to make the covered articular prosthesis; once the covered articular prosthesis is obtained, the device 1 can be disposed of without cleaning and without the need to restore its initial sterile condition.

Preferably, the shell 2 defining the covering chamber 3 is made entirely of single-use material. The shell 2 is, therefore, disposed of at the end of its use, that is, after the covered articular prosthesis has been made.

The device 1 enables a covering chamber 3 to be obtained that is non-reusable and disposable after use. Materials of a various type, preferably polymeric materials such as polypropylene, can be used in the manufacture of device 1 and, in particular, the shell 2.

Considering the covering chamber 3 again, inside the device 1, the covering chamber 3 is shaped as a cavity suitable for receiving the articular prosthesis 11 in addition to a quantity of the substance used, to allow partial or total covering of the articular prosthesis 11.

The covering chamber 3 preferably has a three-dimensional extension similar to that of the articular prosthesis 11, allowing it to be covered without substantially altering its external profile. The term "external profile" refers to the shape of the articular prosthesis 11 or of a section thereof, defined respectively by the entire external surface of the articular prothesis 11 or by a portion of said external surface.

Shells 2 and, consequently, covering chambers 3 of a wide variety of shapes and sizes, which are suitable for receiving, in whole or in part, respective articular prosthesis 11 of different types can be provided.

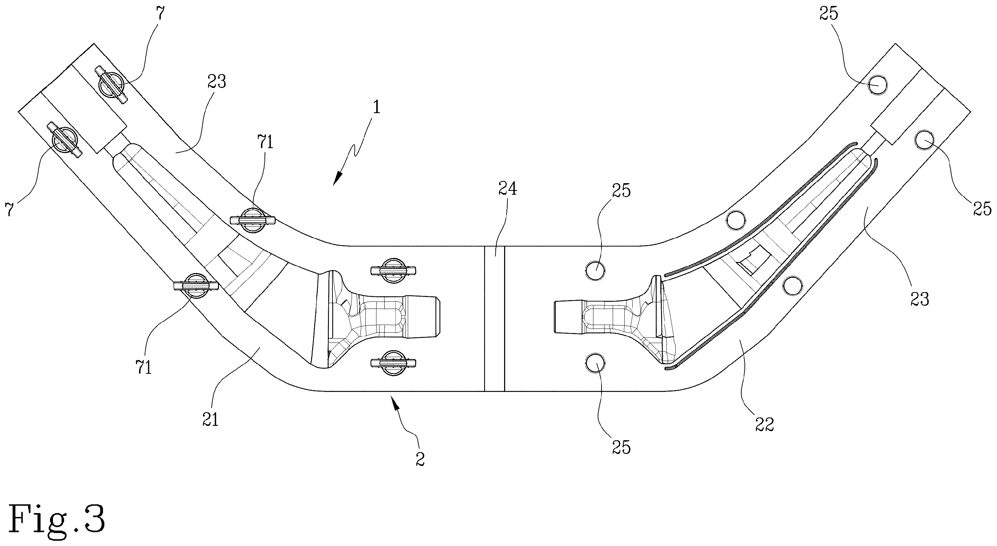

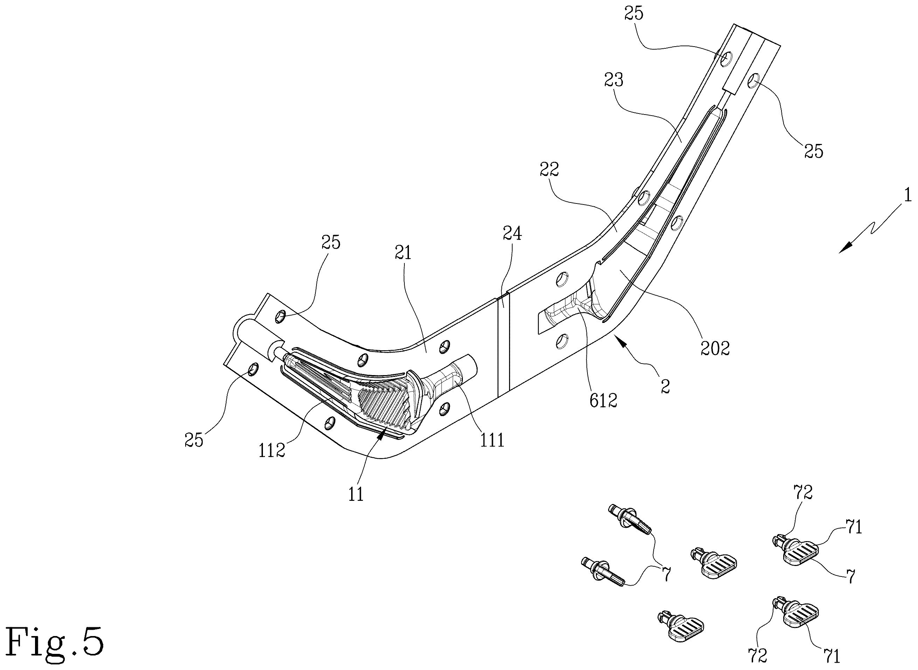

The shell 2 preferably has an inner surface 20 delimiting an elongated covering chamber 3 for making a covered stem prosthesis 10: for example, a covering chamber 3 with an elongated shape can be used for making a covered femoral stem or a covered humeral stem, starting respectively from a femoral prosthesis 11 and a humeral prosthesis (Figures 1-7).

In this case, the injection opening 4 is preferably located in a distal portion of the covering chamber, corresponding to a stem portion of the articular prosthesis 11.

Alternatively, the shell 2 may have an inner surface 20 delimiting a covering chamber 3 shaped to receive, in part or completely, a tibial plate for making a covered tibial plate which can be used in a knee prosthesis; further, alternatively, the shell 2 may have an inner surface 20 delimiting a covering chamber 3 shaped to receive, in part or completely, a tibial component for making a covered tibial component, which can be articulated with the covered tibial plate to make a fully covered knee prosthesis. The latter embodiments of the invention are not shown in the accompanying drawings. The shell 2 may have a second opening 8, shown in Figure 8, for putting the covering chamber 3 in communication with an outside environment, to allow the articular prosthesis 11 to be placed in the covering chamber 3 with an end portion protruding from the covering chamber 3.

The shell 2, having the second opening 8, allows a covered articular prosthesis to be obtained with the covering made of the desired substance only on the portion of articular prosthesis 11 inserted in the covering chamber 3. The end portion protruding from covering chamber 3 will consequently not have a covering made of said substance.

For example, the second opening may be located above the covering chamber 3, to allow the placement of a stem prostheses 11 with an end portion protruding from the shell 2 and a stem portion 112 positioned inside the shell 2. The end portion may correspond to a neck portion 111 or a stem portion 112 proximal to the neck portion 111 of the articular prosthesis 11. The femoral stem thus obtained comprises the articular prosthesis 11 having the neck portion 111 uncovered and the stem portion 112 covered by the covering.

Advantageously, this latter embodiment of making the device 1 allows the covered stem prostheses 11 to be made having different shapes, in particular a plurality of stem prostheses 11 having different inclinations and neck lengths 111. Further, the embodiments described allow the covering of the articular prostheses 11 to be made with different stem lengths 112. A same device 1 can, therefore, be used for covering various types of articular prostheses 11.

In this embodiment, the first opening 4 for injection of medical cement can be positioned in various positions of the device 1.

Preferably, as shown in Figure 8, the first opening 4 for injecting cement is located to the side of the covering chamber 3.

Even more preferably, considering a covering chamber 3 for a stem prosthesis11, the first opening 4 for injection of the cement is located at the portion of the covering chamber 3 intended to receive the stem portion 112 adjacent to the neck portion 111. The injection of the cement therefore takes place at or at the base of the neck 111 of the stem prosthesis 11 which is to be covered. The cement can therefore flow until it fills the covering chamber 3, covering the entire stem 112.

The device 1 also has a connection channel 9 between the covering chamber 3 and the outside environment, to allow the escape of air bubbles during the injection and the solidification of the medical cement inside the covering chamber 3.

The channel 9 can be positioned in various ways relative to device 1.

As can be seen in Figure 8, the channel 9 can be located opposite the first and second openings 4, 8, considering the longitudinal extension of the covering chamber 3. This positioning of channel 9, in particular opposite the first opening 4 for injection of the cement, is particularly effective in releasing any air bubbles formed during the injection and solidification of the cement. The device 1 may further comprise an insert 12 which can be inserted into the covering chamber 3. The insert 12, which is shaped in various ways, can be positioned inside the covering chamber 3 to modify the internal shape of the covering chamber 3 or to reduce at least one dimension of the covering chamber 3.

Advantageously, thanks to the insert 12, the covering chamber 3 can be adapted to specific shape or dimensional requirements. These needs can be met by changing the shape and dimensions of the covering chamber 3 by means of the insert 12.

The device 1 comprising the insert 12 is, therefore, highly adaptable to different prostheses 10 and to the needs, for example anatomical needs, of the patient.

Several inserts 12 can be provided. Inserts 12 having shapes of various types can be used to modulate and create the desired covering on the articular prosthesis 11 which is to be covered.

In the embodiment of Figure 8, the device 1 has an insert 8 of substantially cylindrical type, intended to be inserted into a distal portion of the covering chamber 3. The insert 12, positioned as shown, causes the covering chamber 3 to be shortened, allowing it to cover articular prostheses 11 having shorter stem portions 112 compared with the originally intended length.

The insert, once in place, occupies a smaller volume within the covering chamber. The cement, once injected, stops at the upper surface 121 of the cylindrical insert 12. The device 1 can, therefore, be used to cover an articular prosthesis 11 having a stem portion 112 of shorter length. Alternatively, by removing or not positioning the insert 12, the same device 1 can be used to cover an articular prosthesis 11 of greater length.

The insert 12 therefore gives device 1 a high operational flexibility and adaptability.

The insert 12 can also have at least one through-hole, intended to serve as a connection between the covering chamber 3 and the outside environment, allowing air bubbles formed in the cement to escape. Thanks to the through-hole, the insert 12, placed in the covering chamber 3, does not prevent the correct and optimal solidification of the cement.

According to an embodiment of the device 1, the shell 2 comprises a first half-shell 21 and a second half-shell 22, which can be coupled to the first half-shell 21 to define the covering chamber 3. In detail, the first half-shell 21 can be specular and symmetrical to the second half-shell 22, with respect to a coupling plane, at which the first half-shell 21 couples with the second half-shell 22, to obtain the shell 2 in a closed configuration and defining the covering chamber 3.

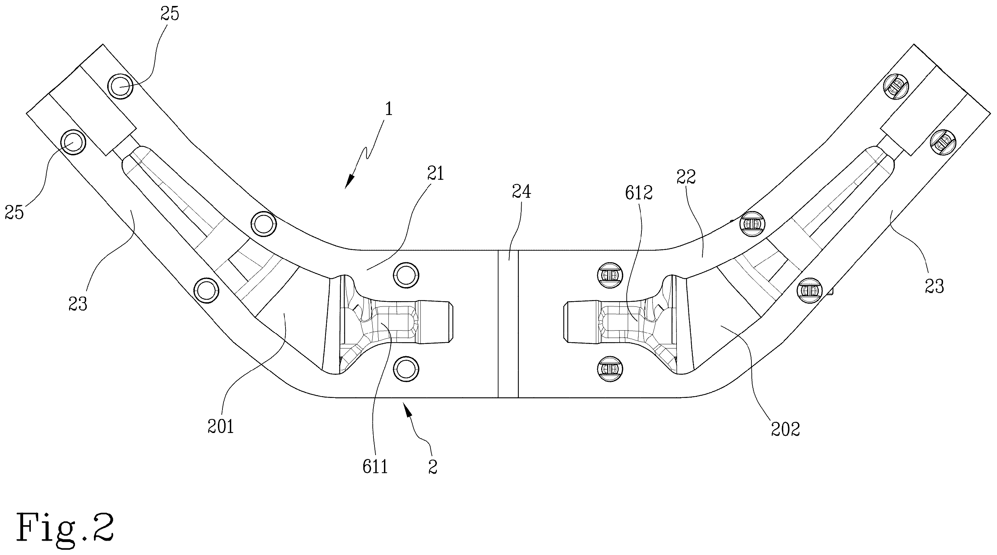

In a first embodiment, illustrated in Figures 2-5, the first half-shell 21 is connected to the second half-shell 22 by means of a connection edge 24. The coupling of the first half-shell 21 to the second half-shell 22 to define the covering chamber 3 takes place by bending the shell 2 at the edge 24 to allow the half-shells 21, 22 to be moved towards each other. Alternatively, the coupling of the first half-shell 21 to the second half-shell 22 to define the covering chamber 3 takes place by breaking the shell 2 at or along the edge 24 to allow the half-shells 21, 22 to be moved towards each other .

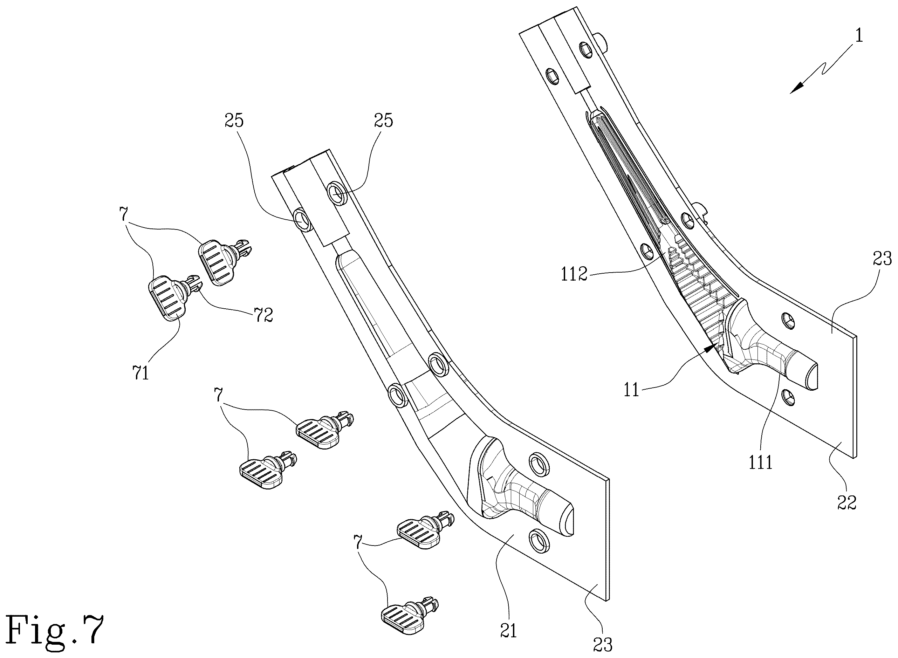

In a second embodiment, illustrated in Figures 6 and 7, the first half-shell 21 and the second half-shell 22 are separated and do not have the connection edge 24.

Further, the first half-shell 21 can be coupled to the second half-shell 22 to define the first opening 4. Further or alternatively, the first half-shell 21 can be coupled to the second half-shell 22 to define the second opening.

The first and second half-shells 21, 22 have respective inner surfaces 201, 202 that can be coupled to define the inner surface 20 and, consequently, the covering chamber 3 which is suitable for receiving the articular prosthesis 11 or part of the articular prothesis 11, together with a quantity of substance to form the covering.

Preferably, the first half-shell 21 and the second half-shell 22 can be coupled by means of respective flanges 23: in other words, the first and the second half-shells 21, 22 comprise respective flanges 23 prepared to allow the mutual moving towards each other of the first half-shell 21 and the second half-shell 22 and to reach the desired mutual position and orientation between the first and second half-shells 21, 22, so as to correctly identify the covering chamber 3.

The flanges 23 are positioned around the perimeter of the respective half-shells 21, 22, while the internal surfaces 201, 202 which define the covering chamber 3 are formed centrally on the respective half-shells 21, 22 so that, following the abutment of the flanges 23, the covering chamber 3 itself is defined inside the closed shell 2.

Moreover, the first and second half-shells 21, 22 may have one or more holes 25 at the flanges 23, said holes 25 being distributed in such a way as to be next to and coaxial with the correct coupling between the first and second half-shells 21, 22. Said holes 25 are engaged in use by closing means 7 to keep the first and second half-shells 21,22 coupled during the injection of the substance and its solidification.

The device 1 may therefore comprise one or more closing means 7 which can be positioned at the first and the second half-shells 21, 22 to keep the first half-shell 21 coupled to the second half-shell 22. The closing means 7 are shaped so as to secure the first half-shell 21 to the second half-shell 22, maintaining their coupling during the introduction and/or solidification of the substance inside the covering chamber 3. Preferably, the closing means 7 comprise a gripping portion 71, which can be gripped by an operator, and a stem portion 72, configured to connect the first half-shell 21 to the second half-shell. In use, the stem portion 72 engages a respective hole 25 of the first and second half-shells 21, 22 in a joined configuration, that is, of the shell 2 in a closed configuration.

The gripping portion 71 is configured to allow the user to insert the closing means 7 into the respective hole 25, causing the first half-shell 21 to couple with the second half-shell 22; after using the device 1, the gripping portion 71 also enables the operator to apply a twist intended to break the stem portion 72 at a weakening present between the stem portion 72 and the gripping portion 71, allowing the first half-shell 21 to be uncoupled from the second half-shell.

According to a further embodiment, the device 1 may comprise centring means 6, to ensure the correct positioning of the articular prosthesis 11 with respect to the covering chamber 3. The centring means 6 are therefore intended to maintain the articular prosthesis 11 or a stretch thereof in the correct position inside the shell 2.

The centring means 6, in other words, both secure the articular prosthesis 11 in the correct position with respect to the shell 2 and maintain the articular prosthesis 11 in said correct position during the injection of the substance and during the solidification of the substance inside the covering chamber 3.

The centring means 6 can be of various types.

In a variant of the invention, the shell 2 has at least one centring seat 6 for the correct positioning of the articular prosthesis 11 inside the covering chamber 3. The centring seat 6 is made by means of a narrowing of the section 61 of the covering chamber 3 which can be engaged by a stretch of the articular prosthesis 11, to constrain said stretch of articular prosthesis 11, obtaining the correct positioning of the articular prosthesis 11 in the covering chamber 3.

In other words, the centring seat 6 is shaped to match a respective stretch of articular prosthesis 11 and can be engaged by said stretch of articular prosthesis 11. The centring seat 6 engaged by the stretch of articular prosthesis 11 prevents any relative movements of the articular prosthesis 11 with respect to the shell 2, caused, for example, by the injection of the substance into the covering chamber 3.

With reference to Figure 1, the shell 2 preferably has a centring seat 6 at a neck portion identified in the covering chamber 3: in this embodiment, the inner surface 20 defines a narrowing of the section 61 of the covering chamber 3 shaped to receive and constrain the neck portion 111 of the stem prosthesis 11.

Considering a shell 2 comprising the first half-shell 21 and the second half-shell 22, the centring seat 6 can be defined by coupling a first centring surface 611 located in the first half-shell 21 and a second surface 612 located in the second half-shell 22. Upon the coupling of the first half-shell 21 to the second half-shell 22, the first surface 611 and the second surface 612 are adjacent and define the narrowing of the section 61.

For example, in order to obtain the correct and stable positioning of the articular prosthesis 11 with respect to the covering chamber 3, the first half-shell 21 can be prepared. The articular prosthesis 11 can therefore be coupled to the first half-shell 21: in particular, the articular prothesis 11 is positioned with respect to the first half-shell 21 in such a way that a relative stretch engages the first surface 611. Finally, the second half-shell 22 is coupled to the first half-shell 21, with the second surface 612 adjacent to, that is, in contact with the first surface 612, to define the centring seat 6 (Figures 4-5 and 6-7).

Preferably, with reference to the accompanying drawings, the narrowing 61 forming the centring seat 6 is shaped to engage a neck portion 111 of the stem prosthesis 11.

Considering a device 1 having the second opening 8 to allow the escape of an end portion of the prostheses 11 from the covering chamber 3 (Figure 8), the centring seat 6 can be made at said second opening 8, that is, the narrowing of section 61 can be located close to the second opening 8.

In other words, the shell 2 has, at the second opening, a narrowing 61 of the section, that is, a neck shaped to match an end portion of the articular prosthesis 11. The stretch of articular prosthesis 11 engages the centring seat 6 thus identified and is constrained by it in the desired position and is functional for the correct making of the covering on the articular prothesis 11.

When the stretch of articular prosthesis 11 engages the centring seat 6 having the narrowing 61 of the section, the articular prosthesis 11, or at least the portion of articular prosthesis 11 located inside the covering chamber 3, is constrained, that is, not movable with respect to the covering chamber 3. In this way, undesired movements of the articular prosthesis 11 within the covering chamber 3 during the introduction of the substance into the covering chamber 3 and during its solidification are avoided.

In the embodiments of the shell 2 comprising the first half-shell 21 and the second half-shell 22, the first half-shell 21 may have the first surface 611, while the second half-shell 22 may have the second surface 612: upon the coupling of the first half-shell 21 to the second half-shell 22, the first surface 611 and the second surface 612 are adjacent and define the narrowing of the section 61 at the second opening 5.

For example, in order to obtain the correct and stable positioning of the articular prosthesis 11 with respect to the moulding chamber, the first half-shell 21 may be prepared. The articular prosthesis 11 can therefore be positioned at the first half-shell 21 such that a stretch of the articular prosthesis 11, in particular an end stretch of the articular prosthesis 11, engages the first surface 611 located in the vicinity of the second opening. Finally, the second shell 22 is coupled to the first half-shell 21, with the second surface 612 adjacent to, that is, in contact with the first surface 612, to define the centring seat 6: in this way, the articular prosthesis 11, in particular the end section of the articular prosthesis 11, is engaged in the centring seat 6 defined by the narrowing of the section 61.

Preferably, the narrowing 61 forming the centring seat 6 at the second opening 8 is shaped to engage a neck portion 111 or a stem portion 112 located at the base of the neck portion 111 of the stem prosthesis 11.

In detail, therefore, the stem prosthesis 11 is coupled to the first half-shell 21, with the neck portion engaging the first surface 611. Therefore, the second half-shell 22 is coupled to the first half-shell 21, defining the narrowing 61 suitable for securing the articular prosthesis 11 in the correct position, that is, with the stem portion 112 positioned inside the covering chamber 3 and the end portion protruding therefrom. The correct positioning is thus maintained during the injection of the medical cement and during the solidification of the substance, making the desired covered stem prosthesis. In this embodiment, where present, the channel 9 for the escape of air bubbles is also defined and identified at the coupling between the first and second half-shells 21, 22, similarly to what has been described and detailed for the first opening 4 for injection of the cement, for the second opening 8 to allow the escape of a portion of the articular prosthesis 11 and for the centring seat 6.

Finally, the shell 2 may have one or more weaknesses; considering a substantially constant thickness in the shell 2, the weaknesses correspond to portions of the shell 2 identifying localised thinning of the above-mentioned thickness, suitable for indicating and defining preferential fracture paths of the shell 2.

The shell 2, in fact, after the solidification of the medical cement inside the covering chamber 3, can be easily disassembled by breaking along the weaknesses, facilitating the release of the covered articular prosthesis. The device 1 can be comprised in a kit for making in the operating room a covered articular prosthesis, which forms a further object of the present invention.

The kit according to the invention comprises a plurality of shells 2 identifying covering chambers 3 of different shapes and sizes, intended to make covered articular prostheses having different shapes and sizes to meet the anatomical needs of a wide range of patients.

For example, shells 2 can be provided for making covered femoral stems of different diameters and lengths, which can be implanted in patients with different functional and anatomical needs.

In the embodiment of the shell 2 comprising the first half-shell 21 and the second half-shell 22, the kit can comprise a plurality of first half-shells 21 and a plurality of second half-shells 22: the first half-shells 21 can be coupled to respective second half-shells 22 to identify covering chambers 3 of different shapes and sizes.

The kit may further comprise a plurality of inserts 12 intended to modify the shape or at least one dimension of the covering chamber 3.

The kit according to the present invention may further comprise the articular prosthesis 11 suitable for replacing, partially or totally, the functionality of a joint. The articular prosthesis 11 can, therefore, be positioned in use, partly or fully, and as described in the previous paragraphs, inside one of the covering chambers 3 identified by the shells 2.

Further, the kit can comprise a supporting element reversibly connectable to the shells 2 to support and keep each shell 2 in a preferred position. In particular, at the end of the introduction of the covering substance into the shell 2, in which the articular prosthesis 11 is positioned, the shell 2 can be placed at the support element: the support element, for example, can comprise a base and a pair of arms suitable for supporting the shell 2 and keeping it in a desired position, allowing the correct solidification of the substance and preventing its escape.

In addition to or alternatively to the pair of arms, the support element may comprise a constraint portion, which can be coupled to the end portion of the articular prosthesis 11 protruding from the shell 2. In this case, the articular prosthesis 11 is supported by the support element during the solidification phase of the medical cement.

Lastly, finally, the covered articular prosthesis made by the method described in the preceding paragraphs also forms an object of the invention. The covered articular prosthesis comprises an articular prosthesis 11 suitable for replacing, partly or totally, the functionality of a joint and a covering of medical cement. The covering covers all or part of the articular prosthesis 11.

Considering the medical cement as the covering substance, preferably supplemented with at least one antibiotic, the covered articular prosthesis can then be implanted in place of an infected prosthesis, in order to restore the functionality of the joint while the infection is treated.

In the preferred embodiment, the covered articular prosthesis is a covered femoral stem, comprising a stem prosthesis 11 covered in a substance, in particular covered in medical cement. Preferably, the stem prosthesis 11 is at least partially covered with medical cement.

The covered stem prosthesis can be implanted at the proximal femur in replacement of a previously implanted and infected stem prosthesis, to treat the onset of infection, while maintaining the functionality of the hip joint.

## Claims

1. A method for making a covered articulated prosthesis in an operating room, comprising the steps of:
Preparing an articular prosthesis (11) designed to replace, partly or completely, the functionality of a joint;
Providing a covering chamber (3);
Positioning at least one portion of the articular prosthesis (11) inside the covering chamber (3);
Introducing a quantity of a substance inside the covering chamber (3), covering the at least one portion of articular prosthesis (11);
Waiting for a predetermined time for the solidification of the substance injected into the covering chamber (3), creating a covering around the stretch of articular prosthesis (11) positioned in the covering chamber (3) to obtain the covered articular prosthesis;
Releasing the covered articular prosthesis from the covering chamber (3) at the end of the predetermined time for the solidification;
Discarding the covering chamber (3) after releasing the covered articular prosthesis.

2. The method according to claim 1, wherein in the step of *Introducing a quantity of a substance inside the covering chamber (3), covering the portion of articular prosthesis (11),* the substance, in liquid form, is introduced into the covering chamber (3) by injection.

3. The method according to any preceding claims comprising the step of:
Ensuring the correct positioning of the articular prosthesis (11) or the portion of articular prosthesis (11) inside the covering chamber (3),
before the step of *Introducing a quantity of a substance inside the covering chamber (3), covering the at least one portion of articular prosthesis (11).*

4. The method according to claim 3, wherein during the step of *ensuring the correct positioning of the articular prosthesis (11) or the portion of articular prosthesis (11) inside the covering chamber (3),* the correct positioning of the articular prosthesis (11) or the portion of articular prosthesis (11) inside the covering chamber (3) is ensured and maintained through centring means (6) positioned at the covering chamber (3).

5. The method according to claim 3, wherein during the step of *ensuring the correct positioning of the articular prosthesis (11) or the portion of articular prosthesis (11) inside the covering chamber (3),* the correct positioning of the articular prosthesis (11) or the portion of articular prosthesis (11) inside the covering chamber (3) is ensured and maintained by engaging at least a stretch of the articular prosthesis (11) in a centring seat (6) identified at the covering chamber (3).

6. The method according to claim 1, comprising the steps of:
Positioning the covering chamber (3) at a supporting element,
before or after the step of *Introducing a quantity of a substance inside the covering chamber (3), covering the portion of articular prosthesis (11).*

7. A device (1) for making a covered articulated prosthesis (10) in an operating room, comprising a shell (2) delimiting internally a covering chamber (3) for the positioning of at least one portion of an articulated prosthesis (11);
wherein the shell (2) has a first opening (4) for putting in communication the covering chamber (3) with an outside environment, to allow the introduction of a substance inside the covering chamber (3) and to cover the at least one portion of articular prosthesis (11), obtaining the covered articular prosthesis (10); and
wherein the shell (2) is partly or totally made from a single-use material.

8. The device according to claim 7, wherein the shell (2) has a second opening for putting the covering chamber (3) in communication with an outside environment, to allow the articular prosthesis (11) to be placed in the covering chamber (3) with an end portion protruding from the covering chamber (3).

9. The device according to any one of claims 7 to 8, wherein the shell (2) comprises at least one centring seat (6) for the correct positioning of the articular prosthesis (11) inside the covering chamber (3).

10. The device according to claim 9, wherein the centring seat (6) is made by means of a narrowing of the section (61) of the covering chamber (3) which can be engaged by a stretch of the articular prosthesis (11), to constrain said stretch of articular prosthesis (11), obtaining the correct positioning of the articular prosthesis (11) in the covering chamber (3).

11. The device according to claim 9 when dependent on claim 8, wherein the centring seat (6) is made by means of a narrowing (61) of the second opening, which can be engaged by a stretch of the articular prosthesis (11), to constrain said stretch of articular prosthesis (11), obtaining the correct positioning of the articular prosthesis (11) in the covering chamber (3).

12. The device according to any one of claims 7 to 11, wherein the shell (2) defines a covering chamber (3) of elongate shape for the positioning of a rod prosthesis (11) to make a covered rod prosthesis.

13. The device according to claim 12, wherein the shell (2) defines a covering chamber (3) with an elongate shape for making a covered femoral rod or a covered humerus rod.

14. The device according to any one of claims 7 to 13, wherein the shell (2) comprises a first half-shell (21) and a second half-shell (22), which can be coupled to the first half-shell (21) to define the covering chamber (3).

15. The device according to claim 14, comprising one or more closing means (7) which can be positioned at the first and the second half-shells (21, 22) to keep the first half-shell (21) coupled to the second half-shell (22).

16. The device according to any one of claims 7 to 15, comprising an insert (12) which can be inserted or positioned in the covering chamber (3), to modify the internal shape or to reduce at least one dimension of the covering chamber (3).

17. A kit for making a covered articulated prostheses in an operating room, comprising:
a plurality of shells (2) of a device (1) according to claim 7, identifying covering chambers (3) of different shapes and sizes.

18. The kit according to claim 17, comprising a plurality of first half-shells (21) and a plurality of second half-shells (22) of a mould according to claim 14, wherein the first half-shells (21) can be coupled to respective second half-shells (22) to identify covering chambers (3) of different shapes and dimensions.

19. The kit according to claim 17 or 18, comprising a supporting element reversibly connectable to the shells (2) to support and keep each shell (2) in a preferred position.

20. The kit according to claim 17 or 18, comprising a supporting element reversibly connectable to an end portion of an articular prosthesis (11) protruding from the shell (2).

21. The kit according to claim 17 or 18, comprising at least one articular prosthesis (11) designed to replace, partly or totally, the functionality of a joint and which can be positioned, in use, partly or entirely inside one of the covering chambers (3).

22. A covered articular prosthesis comprising an articular prosthesis (11) designed to replace, partly or totally, the functionality of a joint and covered, in whole or in part, by a covering substance,
wherein the articular prosthesis is made by following the method according to claim 1.

23. A covered femoral rod comprising a rod prosthesis (11) designed to replace, partly or totally, the functionality of a hip joint and covered, completely or partly, by a covering substance,
wherein the covered femoral rod is made using the method according to claim 1.
